# EUROPEAN PATENT APPLICATION

(11) **EP 1 892 303 A1**
(43) Date of publication of application: **27.02.2008**
(21) Application number: 06076609.4
(22) Date of filing: 22.08.2006
(51) Int. Cl.: C12Q 1/68, G01N 33/574

(54) **Methods for identifying therapeutical targets in tumors and for determining and targeting angiogenesis and hemostasis related to adenocarcinomas of the lung**

(71) Applicant: Fraunhofer-Gesellschaft zur Förderung der angewandten Forschung e.V., 80686 München (DE)
(72) Inventor: Salinas-Riester, Gabriela, Dr., 37073 Göttingen (DE); Borlak, Jürgen, Dr., 31275 Hannover (DE)
(74) Representative: Baumbach, Friedrich

(57) **Abstract**

The problem solved by the invention resides in making available facile and efficient methods for identifying therapeutic tumor targets. The methods of the invention relate to identifying therapeutic tumor targets and the use of these targets in diagnosis, monitoring and therapy of tumors, in particular, advanced stage tumor malignancies.

The invention, further, relates to the use of such targets for identifying substances useful in diagnosis, monitoring and therapy of tumors, in particular adenocarcinomas of the lung. Areas of application are biology, biochemistry, biotechnology, medicine, medical technology as well as other fields of the life sciences. Specifically, the present invention relates to methods for identifying therapeutical targets in tumors, comprising the steps of
- isolating RNA (1) from the tissue of the tumor;
- determining for the isolated RNA (1) a gene expression profile (2) of at least two genes, wherein at least one gene (3) is coding for a VEGF activity modulator and at least one gene (4) is coding for a hemostatic factor by screening the presence of mRNA coding for the factors to be screened and by determining the levels of expression of thereof;
- determining the changes of expression of the at least two genes screened by the gene expression profile (2) in comparison with healthy tissue or with an early stage tumor; and
- identifying the therapeutical target as a hemostatic factor, being
(a) upregulated or downregulated,
if parallely
(b) the VEGF acitivity modulator is downregulated or mainly non changed,
in the gene expression profile (2) in comparison with healthy tissue or with an early stage tumor.

## Description

The invention relates to a method for identifying therapeutical targets in tumors, in particular in advanced stage tumor malignancies, the use of novel targets for identifying, determining, and targeting angiogenesis and hemostasis related to adenocarcinomas of the lung, and the use of the therapeutical targets identified for screening and determining means and/or drugs, and means and drugs for identifying, labeling and treating adenocarcinomas of the lung. Areas of application are the life sciences: biology, biochemistry, biotechnology, medicine and medical technology.

Tumor growth and metastasis are dependent on sustained angiogenesis. The switch to an angiogenic phenotype occurs early in tumor development as a result of genetic changes. This switch is often characterized by increased expression of angiogenic proteins such as VEGF and their receptors. VEGF is one of the major angiogenic growth factors which selectively induce activation, migration, proliferation and tube formation in endothelial cells *in vitro.*

Over the past several years, various compounds have been developed to inhibit secreted proteins such as the much studied VEGF-signaling, which clearly plays a role in tumor angiogenesis. VEGF ligands and its receptors are therefore regarded as potential candidates.

To date, though, none of these compounds have demonstrated convincing efficacy in human therapies (Reese et al. Prostate J 3:65-70 (2001); Nygren & Larsson. J Intern Med 253:46-75 (2003); Emanueli & Mededdu. Arch Mal Coeur Vaiss 97:679-87 (2004); Dali et al. J Natl Cancer Inst 95:1660-73 (2003); Johnson et al. Clin Oncol 22:2184-91 (2004))

Hence, the identification of novel targets, allowing to control and to disturb tumor development, is of great importance. Systematic methods for a goal-directed identfication of targets in tumors and means directed against said targets are urgently needed but, however, are still a great challenge.

The aim of the present invention is therefore to make available an easy and efficient method for identifying therapeutical targets in tumors, in particular in advanced stage tumor malignancies, the use of novel therapeutical targets identified by the method for screening and determining beneficial means and/or drugs, and means and drugs for identifying, determining and treating angiogenesis and hemostasis related to adenocarcinomas, in particular of advanced stage tumors of the lung. To this end, the implementation of the actions and embodiments as described in the claims provides appropriate means to fulfill these demands in a satisfying manner.

Thus, the invention in its different aspects and embodiments is implemented according to the claims.

The inventive method for identifying therapeutical targets in adenocarcinomas is based on the surprising finding that VEGF-signaling is paradoxically repressed in tumors, in particular in advanced stage tumor malignancies, such as in late stage adenocarcinomas of the lung. In fact, the expression of VEGF itself, several cytokines considered as inducers of VEGF and specific VEGF tyrosine kinases receptors were found to be significantly repressed. This astonishing finding is now carefully considered for selecting suitable targets for cancer therapy according to the inventive method.

In principle, expression of hemostatic and/or proangiogenic factors is studied according to the inventive method, such as by reverse transcription polymerase chain reaction or by gene chip analysis, by Western blotting, by histopathology or hematology techniques.

The method acording to the invention for identifying therapeutical targets in tumors, in particular in advanced stage tumors, particularily advanced stage tumor malignancies, such as late stage adenocacinomas of the lung may be, comprises the steps of
- isolating a RNA sample, in the following also designated as "RNA (1)", from the tissue of a mammalian tumor, such as a human or murine tumor may be,
- determining for the isolated RNA (1) a gene expression profile, also termed as "gene expression profile (2)" subsequently, of at least two genes, wherein at least one gene (3) is coding for a VEGF activity modulator and at least one gene (4) is coding for a hemostatic factor by screening the presence of mRNA coding for the factors to be screened and by determining the levels of expression of thereof,
- determining the changes of expression of the at least two genes screened by the gene expression profile (2) in comparison with healthy tissue or with tissue of an early stage tumor in particular of the type of organ wherein the tumor is formed.
- identifying the therapeutical target as a hemostatic factor screened being significantly upregulated (induced) or downregulated (repressed) in the gene expression profile (2), e.g. being enriched or non detectible, in comparison with healthy tissue or with an early stage tumor, if parallely the VEGF acitivity modulator is significantly downregulated or mainly non changed in the gene expression profile (2) in comparison with healthy tissue or with an early stage tumor.

Tissues are preferably resected or isolated from the tumors, in particular of human patients, by standard procedures or other known methods of resection or isolation. According to the invention the term "tissue" comprises cellular material of tumors and organs in all different forms, e.g. tissue dices, cells, cell compartments, or homogenisate of tissues, cells or cellular compartments. For example, resected tissues can be lysed as a whole or be seperated in cells, the latter providing the beneficial possibility of amplifyng the cellular material of the tumor in cell culture before cell lysis. Hence, in principle only one cell of the tumor tissue is sufficient for receiving approriate amounts of RNA to be isolated. Tissues used are preferably resected or isolated from advanced stage tumor malignancies, such as late stage adenocarcinomas may be, in particular of the breast, colon, lung, stomach, prostate, pancreas or cervix.

Preferably, the RNA is isolated from the tissue of an advanced stage tumor malignancy of the breast, colon, lung, stomach, prostate, pancreas or cervix, in particular of a human being. The latter provides the favourable characteristic that therapeutical targets can be individually identified for a patient, allowing a tailored diagnostic and treatment of the patient. More preferably, the RNA is isolated from a late stage adenocarcinoma, in particular from an advanced stage tumor of the lung.

According to the invention, the term "early stage" in particular concerncs the initiation stage of a tumor, the term "advanced stage", particularily concerns the progression stage of a tumor, in the three stage process of carcinogenesis as, for example, defined by Pitot et al. (Supramol Struct Cell Biochem 17:133-46 (1981)). The term "late stage" in particular concerns the ultimate stage of a tumor progression stage.

The RNA to be isolated can be purified from the tissues, in particular mammalian tissue, more particular human tissue, according to standard procedures or other known methods, such as by using RNA isolation kits. According to the invention, standard RNA isolation procedures have the advantegous characteristic, that they result in a solution including the transcriptome of the respective tissue, i.e. the set of all mRNA molecules (or *transcripts*) in one cell or a population of the tissue cells for the given set of environmental circumstances, thus allowing the detection of all transcripts of hemostatic and angiogenic factors being present in the tissue at a time.

The hemostatic factors, whose presence is screened according to the inventive method are mammalian, e.g. human and/or murine, preferably human, gene products, in particular proteins being expressed in the species, in particular in the tumor of the species, whose RNA (1) is investigated, regulating the vasoconstriction, primary and/or secondary hemostasis, clot formation or clot lysis, in particular fibrinolysis, coagulation and platelets, and/or are regulating angiogenesis, in particular sprouting or intussusceptive angiogenesis such as of endothelian cells, e.g. HGF activity modulators involved in HGF signaling. Accordingly, the hemostatic factors to be screened are easily chosen from standard databases, from literature, such as review articles or chapters may be, or from information published elsewhere. Preferably, the expression of at least one mammalian, in particular human and/or murine, preferably human, gene selected from the group of genes in Table 3 and/or of HGFA, Sema4A, and/or plxnb2 is determined. For example, the presence of mRNA coding for human and/or murine, preferably human, HGFA, Sema4A, plxnb2, Kng1, FVII, VWF, TFPI, Tna, Plat, serpine2, and/or thrombomodulin is determined. Preferably, the presence of at least two, more preferably of at least three hemostatic factors is determined.

According to the invention, in the isolated RNA (1) the presence of mRNA sequences coding for one or more mammalian, e.g. human and/or murine, preferably human, angiogenic, in particular proangiogenic, factors, particularily coding for at least one VEGF activity modulator, such as mRNA coding for VEGF, coding for a cytokine inducing VEGF or coding for a VEGF tyrosine kinase receptor is determined. Preferably, the expression of at least one mammalian, in particular human and/or murine, preferably human, gene selected from the group of genes in Table 1 is determined. More preferably, the presence of mRNA coding for Vegfa, Vegfc, Figf, Fit1, Kdr, Tie1, and/or Tek, is determined. In particular, the presence of mRNA coding for Fgfr4, Fgfr3, Edg6 and/or Edg1, being specified in Table 1, is determined, the latter representing genes being expressed in normal tissues and absent in advanced stage adenocarcinoma of the lung, thus providing further advantegous characteristics for different aspects of the inventive method. Preferably, the presence of at least two, more preferably of at least three angiogenic factors is determined.

For the isolated RNA (1) a gene expression profile of at least two genes coding for hemostatic and/or angiogenic factors, in particular hemostatic and/or proangiogenic factors as specified above, preferably hemostatic and/or proangiogenic factors selected from the group of genes in the Tables 1-3, e.g. coding for (a) a VEGF activity modulator and for (b) HGFA, Sema4A, plxnb2, Kng1, FVII, VWF, TFPI, Tna, Plat, serpine2, and/or thrombomodulin is determined by screening the presence of mRNA coding for the factors to be screened and by determining the levels of expression of thereof. According to the invention the gene expression profiling of the at least two genes comprises the screening and determining of one gene (3) coding for a VEGF activity modulator, in particular coding for a VEGF modulator as specified above. The term "level of expression" according to the invention in particular concerns the amount of mRNA transcripts being present in a transcriptome or a value derived of thereof.

The expression profiles to be determined according to the invention are performed by standard procedures of expression profiling or other known methods for parallely determining the presence of several different mRNA molecules in an isolated RNA sample, e.g. by PCR methods for amplifying and/or synthesizing oligonucleotides and by determination (qualitatively and/or quantitatively) of the such produced oligonucleotides using (gel) electrophoretic separation or array techniques and subsequently registering, imaging, estimating, and/or calculating the present amounts of angiogenic and/or hemostatic factors in the samples tested (levels of expression). For imaging purposes all possible and standard imagers and scanners for vizualizing separated or spatially enriched oligonucleotides, e.g. a Lumi-imager or a microarray scanner, are suitable, thus allowing an easy adaption of the invention to different laboratory equipments.

According to the inventive method the levels of expression determined are used for quantifying the changes of expression, e.g. as fold change, of the at least two genes screened in comparison with their expression in healthy tissue or in tissue of an early stage tumor, in particular of the sort of organ, wherein the tumor is formed, by standard procedures or by any other known method for determining changes of expression. Preferably a standard or reference is used for determining the changes of expression, in particular the level of gene expression of at least one of the genes to be screened derived from a gene expression profile for a healthy tissue or for an early stage tumor of the organ, wherein the tumor is formed, is used. For this purpose, the levels of expression of the at least two genes coding for the factors screened are preferably normalized with respect to the levels of expression of the respective genes in healthy tissue or in an early stage tumor of the organ, wherein the tumor is formed, in particular by using the standard for normalizing.
The standard can for example be either determined parallely and/or analogically to the inventive method or a respective value determined before is used.

More preferably, the standard comprises values for the expression levels of at least one VEGF activity modulator and of at least one hemostatic factor screened and/or a value for the expression level of a gene being non changed, in particular having a value of less than (<) 1.5 fold change and greater than (>) -1.5 fold change in the preferably visible/solid tumor in comparison with the respective healthy tissue and/or in comparison with the early stage tumor, such as mammalian, in particular human and/or murine, preferably human, HGF or β-Actin may be.
The changes of expression of the factors screened are preferably compared with the at least one standard.

The term "upregulated or downregulated" and "induced or repressed", respectively, particularily concerns a change of expression being > 1.5 fold change or < -1.5 fold change in the preferably advanced stage tumor in comparison with the respective healthy tissue and/or in comparison with the early stage tumor and/or in comparison with the standard. In this regard, the term "downregulated" relates to signals and/or values, in particular changes of expression, being significantly, particularily with a fold change < - 1,5 fold change, less than a value determined for at least one control (standard), which is preferably determined parallely to the gene expression profile (2) or is included therein. For example, the level of expression of HGF is determined, such as dislosed in Table2b, and chosen as the standard (4) or any other control being non differentially expressed in tumors in comparison with healthy tissue, such as β-Actin may be and the change of expression of one of the at least two factors to be screened, such as VEGF may be, is found in the range of the change of expression of HGF and/or β-Actin, including the deviation around the average value, if replicates (e.g. n = 3) are determined for HGF and/or VEGF, then VEGF is mainly non changed in comparison with the standard (4).

The term "mainly non changed" relates to signals and/or values, in particular of changes of expression, being in the range of a value determined for at least one control (standard), which is preferably determined parallely to the gene expression profile (2) or is included therein. For example, the level of expression of HGF is determined, such as dislosed in Table 2a or Table2b, and chosen as the standard or any other control being non differentially expressed in tumors in comparison with healthy tissue, such as β-Actin may be and the change of expression of one of the at least two factors to be screened, such as VEGF may be, is found in the range of the change of expression of HGF and/or β-Actin, including the deviation around the average value, if replicates (e.g. n = 3) are determined for HGF and/or VEGF, then VEGF is mainly non changed in comparison with the standard (4).

In the same manner, any other values or levels, e.g. the level of expression, deduced from the signal(s) or signal intensities is approriate for classifying the modulator and/or factor as being upregulated or downregulated. In this context preferably a (molecular) standard, e.g. a transcript, such as of HGF or β-Actin, being detectable in the tumor and the respective healthy tissue is parallely used or determined for standardizing the results received.

The afore mentioned principle of pairwisely evaluating signals or values is also analogously perfomed for the correlation or comparison of proteins and fragments of thereof, as being described beneath.

The gene expression profiles determined according to the invention are preferably mutually compared with the standard and/or normalized to the standard, by regular procedures or other known methods for comparing and/or normalizing the results of gene expression profilings.

Preferably, the standard includes a value, in particular the level of gene expression of one of the genes to be screened, derived from a gene expression profile for a healthy tissue or for an early stage tumor of the organ, wherein the tumor is formed. Preferably, the standard includes signals and/or values for the expression levels of a VEGF activity modulator and of a hemostatic and/or proangiogenic factor screened, in particular being determined parallely to the gene expression profile (2) or determined before.

According to the inventive method the therapeutical target as a hemostatic factor screened being significantly upregulated or downregulated in the gene expression profile (2), e.g. being enriched or non detectible, in comparison with healthy tissue or with an early stage tumor, if parallely the VEGF acitivity modulator is significantly downregulated or mainly non changed in the gene expression profile (2) in comparison with healthy tissue or with an early stage tumor.

The gene expression profile (2) is thus pairwisely compared with the standard and the results are joint and/or correlated, so that the overall expression status of the angiogenic and/or hemostatic factors screened can be determined as being
(a) upregulated or downregulated in the sample RNA (1) or
(b) mainly non changed or downregulated in RNA (1)
in comparison with healthy tissue and/or an early stage tumor, in particular of the organ, wherein the tumor is formed.

Since it was found in the work leading to the invention, that the levels of VEGF modulators are repressed and/or downregulated in advanced stage tumor tissues, whereas hemostatic factors are reciprocally induced and/or upregulated, also the level of expression of mRNA coding for gene (3), in particular being determined according to the inventive method, can be used as the standard, thus further reducing the work expense in practice.

In practice, if microarrays are used for the inventive method , the levels of expression of the factors screened are determined for a late stage tumor and the fold of change (FC) is used as filter: the fold of change of the expression levels of a gene (probe set) is examined between two conditions (e.g. tumor and healthy tissue, or late stage tumor and early stage tumor). If the ratio is above or below a predefined cut-off threshold (e.g. 1.5-, two- or four-fold change), these genes are declared to be differentially expressed, and are selected for further analysis.

Only if the VEGF activity modulator(s), e.g. Vegfc and/or Tie1, is repressed (e.g. -1.5, -2, or -4 fold change) and the hemostatic and/or angiogenic factor(s) screened (e.g. Hgfac and/or Kng1) is induced, then the hemostatic and/or angiogenic factor(s), e.g. Hgfac and Kng1, is/are identified as therapeutical target(s) according to the invention.

Preferably, each level of expression is determined several times (e.g. in triplicate) and a statistical test is used for filter, e.g. a regular parametric t-test and/or a non-parametric Kruskal-Wallis rank sum test or other methods usable in conjunction with permutation tests.

If, in praxis, a qRT-PCR is used for identifying therapeutical targets, then the fold change of levels of expression of the at least one VEGF activity modulator, e.g. VEGFa, and of the at least one angiogenic and/or hemostatic factors screened, e.g. cMet and/or Sema4A, is determined, accordingly. If the FC of the VEGF activity modulator, e.g. VEGFa, is above or below the cut-off threshold (e.g. FC > 1.5 or FC < -1.5), then the FC value is compared with the FC of a gene expression level usually unchanged in tumors, e.g. of HGF or β-Actin. If the range of the FC value +/- its' error or deviation (e.g. mean FC of VEGFa +/- its' error) is in the range of the usually unchanged gene expression level (e.g. mean FC of HGF +/- its' error) than the VEGF activity modulator is determined as mainly non changed. In the uniform manner, the FC (mean +/- error or deviation) of the hemostatic and/or angiogenic factor is compared with the FC (mean +/error or deviation) of the known unchanged expression level, e.g. of HGF, and if it is significantly higher (e.g. FC being higher and having no overlap in its' error range with the error span of the FC of the usually unchanged gene expression), then the angiogenic and/or hemostatic factor(s),), e.g. cMet and/or Sema4A, is/are identified as therapeutical target(s) according to the invention.

In yet another aspect, the expression profiling comprises the synthesis of a cDNA library (5) derived of the isolated RNA (1) and preferably the synthesis a cDNA library (6) derived of RNA of the healthy tissue or of the early stage tumor of the organ, wherein the tumor is formed, by a reverse transcription polymerase chain reaction (RT-PCR), enabeling an easy first strand reaction, and/or by quantitative RT-PCR.

Yet another aspect of the invention concerns the gene expression profiling which further comprises the steps of amplifying cDNA sequences of the hemostatic and/or proangiogenic factors to be screened by a PCR, in particular by using a thermocycler, wherein the cDNA library (5) and optionally the cDNA library (6) is used in a mixture with synthetic primers being, at least in parts, complementary with the hemostatic and/or proangiogenic factors to be screened, the primers being preferably selected from the group of the primers as described below (Methods), and separating the amplified cDNA sequences by gel electrophoresis of the PCR reaction mixtures and vizualizing the separated PCR products. This aspect allows a straightforward approach for fulfilling the invention in a specific sensitive manner.

Preferably, for vizualizing the separated PCR products, such as by standard procedures, iabeied synthetic primers are used in the PCR, or a substance, in particular a dye such as ethidium bromide may be, intercalating in double stranded oligonucleotides, is used for labelling the PCR products, thus enabeling an easy identification of oligonucleotides.

A further aspect of the inventive method relates to the gene expression profiling being implemented by the steps of synthesizing at least one cRNA library (6) derived of the cDNA library (5) and optionally synthesizing a cRNA library (7) derived of the cDNA library (6) by second strand cDNA synthesis and by *in vitro* transcription of the double stranded cDNA, producing a RNA fragment library (8) derived of the cRNA library (6) and optionally producing a RNA fragment library (9) derived of the cRNA library (7) by hydrolytic cleavage into RNA fragments, e.g. by metal-induced hydrolysis into RNA fragments of the length of 35-200 bases, performing a hybridization assay by incubating an oligonucleotide array (10) including spatially addressed solid phase bound oligonucleotide sequences coding for the at least two hemostatic and/or angiogenetic factors to be screened with a solution of dissolved cRNA fragment library (8) and optionally performing a hybridization assay by incubating an oligonucleotide array (11), being at least in parts identical to array (10), including spatially addressed solid phase bound oligonucleotide sequences coding for the at least two hemostatic and/or angiogenetic factors to be screened with a solution of dissolved cRNA fragment library (9) and scanning the hybridization patterns of the oligonucleotide array (10) and, optionally, array (11). This aspect of the invention allows a fast and efficient detection and/or evaluation of a large number of different hemostatic and/or angiogenic factor transcripts, in particular if labelled ribonucleotides, such as biotin labelled ribonucleotides may be, are used for the *in vitro* transcription, and/or oligonucleotide microarrays , e.g. DNA microarrays, are used for performing the hybridization assays.

Yet a further aspect of the invention concerns a method, wherein HGFA, Sema4A, plxnb2, Kng1, FVII, VWF, TFPI, Tna, Plat, serpine2, and/or thrombomodulin H is/are determined/identified as the target, in particular two factors, preferably three factors, most preferably more than three factors of thereof are identified, thus allowing to proof and support the invention in its different embodiments, e.g. if methods not yet known are used for putting the invention into practice. In particular, the invention is preferably put into practice if genes and/or gene products of HGFA, Sema4A, plxnb2, Kng1, FVII, VWF, TFPI, Tna, Plat, serpine2, and thrombomodulin are identified.

Another aspect of the invention concerns the use of one or more therapeutical targets, in particular of targets being identified according to the inventive method as described, for identifying, determining, and targeting angiogenesis and hemostasis related to adenocarcinomas of the lung, such as identifying diagnostic markers in bodyfluids or tissue, determining the presence of mRNA or proteins in tumor cells, and targeting mRNA or proteins by therapeutical means may be. The use according to the invention, comprises the steps of isolating a biological sample (12) from a mammalian organism, such as from a human and/or mouse, preferably from a human patient, suffering from lung cancer or from an organism to be tested for its susceptibility to lung cancer, and determining the level of mammalian, in particular human and/or murine, preferably human, HGFA, Sema4A, plxnb2, Kng1, FVII, VWF, TFPI, Tna, Plat, serpine2, and thrombomodulin or of fragments of thereof or of a selection of thereof in the biological sample (12) by screening the presence of said proteins or of fragments of thereof or of mRNA coding for the same.

Preferably, the inventive use comprises the steps of isolating a biological sample (13) from a healthy mammalian organism, in particular from a human being, determining the level of HGFA, Sema4A, plxnb2, Kng1, FVII, VWF, TFPI, Tna, Plat, serpine2, and thrombomodulin or of fragments of thereof or of a selection of thereof in the biological sample (13) by screening the presence of said proteins or of fragments of thereof or of mRNA coding for the same and/or pairwisely comparing the gene expression profiles determined for the isolated biological samples (12) - (13) by correlating the levels measured.

More preferably, the isolated biological sample used is a tissue, a cell, a cellular compartment, total RNA, total protein or a body fluid, in particular being isolated from a lung adenocarcinoma or from the blood of the organism.

For the inventive use preferentially an organism suffering from lung cancer is selected or the organism to be tested for its susceptibility to lung cancer is a transgenic animal, in particular a rodent, such as a c-myc mouse may be.

According to the inventive use the level is preferably determined by gene expression profiling, by western blotting or by histopathology. In particular, the gene expression profiling comprises the steps of (a) synthesizing a cDNA library derived of the isolated RNA by RT-PCR, (b) synthesizing a cRNA library, derived of the cDNA library by second strand cDNA synthesis and *in vitro* transcription, wherein preferably labelled ribonucleotides, such as biotin labelled ribonucleotides may be, are used of the double stranded cDNA, (c) producing a RNA fragment library derived of the cRNA library by hydrolytic cleavage into RNA fragments, (d) performing a hybridization assay by incubating an oligonucleotide array, preferably a microarray, including spatially addressed solid phase bound oligonucleotide sequences coding for the at least two oligonucleotide sequences to be screened or for parts of thereof or for sequences being complementary of the same, with the cRNA fragment library and/or (d) scanning the hybridization pattern of the oligonucleotide array.

In another preferable embodiment of the inventive use, antibodies directed against HGFA, Sema4A, plxnb2, Kng1, FVII, VWF, TFPI, Tna, Plat, serpine2 and/or thrombomodulin are used.

In yet another preferential embodiment of the inventive use, one or more genes and/or one or more gene products of thereof selected from the group of HGFA, Sema4A, plxnb2, Kng1, FVII, VWF, TFPI, Tna, Plat, serpine2, thrombomodulin and/or their mutants and/or variations and/or parts thereof and/or derived molecules is used to screen for and to identify drugs targeting angiogenesis and hemostasis related to tumor malignancies of the lung, in particular drugs against adenocarcinoma of the lung.
In particular, one or more genes selected from the group of HGFA, Sema4A, plxnb2, Kng1, FVII, VWF, TFPI, Tna, Plat, serpine2, thrombomodulin and/or their mutants and/or variations and/or parts thereof and/or related molecules and/or their gene products and/or derived structures are incubated with a compound to be tested and changes in the expression of said genes and/or derived sequences and/or the function of said gene products and/or derived structures are determined. Preferably, drugs regulating the expression of one or more of said genes and/or the function of one or more of said gene products and/or their derived molecules and are employed for the production of means for treatment of tumor malignancies of the lung, in particular for the treatment of a adenocarcinoma of the lung.

According to the inventive use, preferably DNA and/or or related molecules encoding one or more of said gene products and/or derived structures are applied, in particular one or more polypeptides, peptides and/or derived molecules having the function of one or more of said gene products, are used.

Yet another aspect of the invention concerns a procedure for identifying, labelling and treating of tumor malignancies of the lung, such as a lung adenocarcinoma may be, wherein a biological or biotechnological system is contacted with a soluble substance; such as an oligonucleotide sequence or antibody may be, having affinity with at least one of the genes selected from the group of mammalian, in particular human and/or murine, preferably human, HGFA, Sema4A, plxnb2, Kng1, FVII, VWF, TFPI, Tna, Plat, serpine2, thrombomodulin and/or their variants and/or parts thereof and/or their mRNA and/or their gene products and/or parts thereof and wherein the soluble substance is linked with a marker.

As the, at least partially, soluble substance in particular oligonucleotides, proteins, peptides or structures derived of thereof are suitable. These have the advantage that they can recognize specifically two or three-dimensional target structures on the molecular level. Beyond that, they provide the favourable characteristic that the recognition usually takes place in aqueous physiologically buffered solutions and leads to a specific association/binding with the targeted structure.
Thereby, monoclonal and/or polyclonal antibodies and/or antibody fragments are particularly suitable, since they are formed as stable highly specific structures, which are, in principle, producible against all possible molecular target structures. In a favourable embodiment of the procedure human and/or bispecific antibodies or human and/or bispecific antibody fragments are used,
In particular, monoclonal antibodies and/or antibody fragments are thereby suitable.
For implementing the invention it is preferred, if the marker according to invention is selected as an element, an isotope, a molecule and/or an ion or is composed of thereof, such as a dye, contrast means, chemotherapeutic agent, radionuclide, toxin, lipid, carbohydrate, biotin, peptid, protein, microparticle, vesicle, polymer, hydrogel, cellular organelle, virus and/or whole cell, in particular if the marker is formed as dye labeled and/or enzyme-labeled secondary antibodies and/or as protein A and/or as protein G or structures derived of thereof.
The linkage between the marker and the substance is favourably chemically, electrostatically and/or over via hydrophobic interactions, such as there is sufficient connection stability for the use of the marked substance for identifying, labelling and treating of metastatic cells, preferably if the linkage is covalent.
For the increase of the sensitivity of the procedure according to invention also the simultaneous use of several substances is in particular suitable, in particular if they comprise two or more substances having each affinity with one of the factors selected from the group of HGFA, Sema4A, plxnb2, Kng1, FVII, serpine2, or their mRNA sequences or their gene products.
In particular, for a specific recognition/identification substances are used, which bind with an affinity above the association constant Ka = 1000 M-1 to the target structure.

For implementing the procedure according to the invention it is favourable to use one of the following methods - PCR, in vitro translation, RT-PCR, gel electrophoresis, Western Blot, Northern Blot, Southern Blot, ELISA, FACS measurement, chromatographic isolation, UV microscopy, immunohistochemistry, screening of solid phase bound molecules or tissues and/or biosensory investigation - whereby by amplification, isolation, immobilization and/or detection and/or by combinations of thereof a particularly simple conversion of the procedure according to invention is made possible for the examined sample, in particular if furthermore a statistic analysis is accomplished.

The procedure according to invention is preferably implemented by using molecules, cells and/or tissue, in particular being immobilized or synthesized on a planar surface, e.g. spatially addressed. on a nitrocellulose or PVDF membrane, or is linked to the cavity of a microtiter/ELISA plate or on the bottom of a cell culture container, in particular on a glass or plastic chip (biochip).
Favourably, as solid phase bound molecules, substances are used, having affinity for at least one and in particular all of the genes HGFA, Sema4A, plxnb2, Kng1, FVII, serpine2 and/or their variants and/or parts of thereof and/or their mRNA and/or their gene products and/or cleavage products derived of the thereofs, polypeptides or peptides
The indentification of target structures, e.g. of hemostatic and/or proangiogenic factors in an immobilized section of tissue or of cells of a cell culture, can take place thereby with arbitrarily marked molecules, which are brought on the surface in solution, e.g. (fluorescence marked/labeled) antibodies.
If a RT-PCR is accomplished, then favourably appropriate oligonucleotide probes and/or primers are used. For implementing the procedure according to invention immobilized molecule libraries, e.g. DNA or antibody libraries are used, which associate with the target structure(s) in solution, e.g. with a cDNA library, a PCR product library or with cells of a secondary tumor.
Substances bound to the molecule libraries are particularly identified by the use of appropriately marked probes, e.g. dye labeled oligonucleotides. If unabeled primary antibodies are used, preferably labeled secondary antibodies are used for detection. Furthermore, the use of other proteins, e.g. enzymes, or streptavidin or parts of thereof is suitable. For the diagnosis of a secondary tumor, in particular by *in vitro* and/or *in vivo* diagnostics, with the help of the procedure according to invention preferably optically (or radiographically) sensitive equipment, is used, e.g. an UV microscope, scanner or ELISA reader, photometer, or szintigrafic equipment, e.g. X-ray gadget are appropriate.

A further aspect concerns the inventive procedure, wherein the biological or biotechnological system used is an organism, a tissue, a cell, a part of a cell, a DNA, a RNA, a cDNA, a mRNA, a cRNA, a protein and/or a peptide and/or a derived structure and/or contains the same, such as cells of a late stage tumor and/or an oligonucleotide library may be.

The biological or biotechnological system employed for the procedure preferably comprises cells of a tumor malignancy of the lung and/or an oligonucleotide library and/or a protein library and/or a peptide library, such as a transgenic animal, e.g. a c-myc mouse, or biological material received from a human adenocarcinoma patient may be.

A further aspect of the invention relates to the use of one or more substances, in particular being identified according to the procedure as decribed, having affinity with genes selected from the group of mammalian, in particular human and/or murine, preferably human, HGFA, Sema4A, plxnb2, Kng1, FVII, VWF, TFPI, Tna, Plat, serpine2, thrombomodulin and/or their mutants and/or variations and/or parts thereof and/or their gene products and/or related molecules of said genes and/or derived molecules of said gene products for preparing a medicament for the treatment of a solid adenocarcinoma, in particular of an advanced stage tumor of the lung.

Yet another preferable aspect of the invention concerns a testkit for identifying and/or determining mammalian, in particular human and/or murine, preferably human, tumor malignancies, in particular advanced stage tumors of the lung, comprising a soluble substance as specified above, for a fast and easy implementation of the invention.

Vascular endothelial growth factor (VEGF) and hepatocyte growth factor (HGF/SF) are two potent mitogens with demonstrated angiogenic activities in human and animal models. Studies based on gene expression profiles indicate that these growth factors exhibit very little overlap in the signal transduction pathway inducing angiogenesis. According to the invention, ways in which angiogenesis is coordinated by hemostatic events during the development of lung adenocarcinomas are outlined.
VEGF-signaling is paradoxically repressed in lung tumors. In fact, the expression of VEGF itself, several cytokines considered as inducers of VEGF and specific VEGF tyrosine kinases receptors were significantly repressed. Evidence is provided that kininogen 1 (Kng1), factor VII (F7), hepatocyte growth factor activator (HGFA) and the receptor of HGF/SF (MET) are key molecules able to participate in switches for turning on angiogenesis in lung adenocarcinomas. Furthermore, the activation of MET occurs through an independent HGF/SF mechanism.

The signaling pathways that mediate angiogenesis during tumor progression are more complex than once believed. In lung adenocarcinoma it is now demonstrated that inhibition of a specific signal transduction pathway will not be sufficient to inhibit neovascularization. In addition, effective inhibition of VEGF angiogenic pathway may lead to selection of cells whose angiogenic activity is mediated through alternative pathways.

Pathways driven from pro- and anticoagulant, fibrinolytic, cell adhesion molecules, extracellular matrix, growth factors, and other endogenous systems participate in the modulation of angiogenesis. Because hemostatic disorders occur early in tumor development as a result of tissue responses to injury, the work leading to invention shows regulatory steps linking hemostasis and angiogenic pathways during tumorigenesis in lung cancer.

The activation of alternative angiogenic pathways such Kininogen (Kng1), Hepatocyte growth factor activator (HGFA) and the MET oncogene (MET) are also regulate by the hypercoagulable state in lung tumors. It has been shown that thrombin promote the activation of pro-HGFA by cleavage following Arg⁴⁰⁷ in the presense of a negatively charged substance. Furthermore, since blood coagulation is often linked to tissue injury, it is reasonable to postulate that plasma pro-HGFA is first activated in local injured tissue by thrombin.

Other features and advantages will become apparent from the following detailed description.

In the following, the concept and proof of the invention is exemplarily shown for adenocarcinomas of the lung but the invention is, in analogy, appropriate to all different forms of tumor malignacies, as described herein.

Tumor growth and metastasis are dependent on sustained angiogenesis. In transgenic mice, the switch to an angiogenic phenotype occurs early in tumor development as a result of genetic changes¹. This switch is often characterized by increased expression of angiogenic proteins such as VEGF and their receptors². VEGF is one of the major angiogenic growth factors which selectively induce activation, migration, proliferation and tube formation in endothelial cells *in vitro.* VEGF exerts its biological effects on endothelial cells through its two major tyrosine kinase receptors, VEGFR1/Flt-1 and VEGFR2/KDR^{3,4}.

Hepatocyte growth factor (HGF/SF) is a mesenchyme-derived mitogen that stimulates cell migration, branching and/or tubular morphogenesis of epithelial and endothelial cells^{5,6}. Deregulation of HGF-signaling by hepatocyte growth factor activator (HGFA) or HGF/SF tyrosine kinase receptor (MET) is frequent in human tumors and is often associated with an aggressive tumor phenotype and consequently with poor prognosis⁶. In physiological conditions, MET activation is a transient event, whereas in tumor cells MET is often constitutively activated⁷. This has to be carefully considered when selecting suitable targets for cancer therapy and HGF/SF and MET are regarded as potential candidates.

Recent studies indicate that VEGF and HGF/SF growth factors may act synergistically to promote angiogenesis in tumorigenesis and *in vivo* experiments demonstrated that the combination of HGF/SF and VEGF increased neo-vascularization in the rat corneal assay greater than either growth factor alone and suggest that a combination therapy targeting HGF/SF and VEGF may provide a more effective strategy for anti-angiogenic therapies^{8,9}. Moreover, it has recently been shown that HGF-signaling drives a genetic program linking cancer to hemostasis ^{10,11}.

According to the invention novel expression patterns of classical and alternative angiogenic pathways and networks that connected angiogenesis to hemostasis during tumor development, is reported. In fact, during tumor growth, the tight regulatory balance between pro- and anti-angiogenic factors is disturbed, resulting in hemostatic disorders.

### Results

By genechips analysis gene expression profiles in histologically defined 12-month old lung adenocarcinomas (n=6) and compared transcripts with a baseline corresponding to normal lung tissues (n=8) were investigated. Early, intermediate and late stage of lung tumors have been investigated. Histologically, a multicentric atypical adenomatous hyperplasia (AAH) with either low and high grade atypia predominantly in alveolar type II cells was observed. The largest adenoma-like nodules put in evidence signs of initial invasive growth, giving rise to a diagnosis of foci of early adenocarcinoma.
High nodule density was observed in intermediate stage as a result of early tumor collisions. This event ended up in a solid confluent tumor mass which occupied the whole lung tumors at an age of 11-month in average (late stage). After statistical analysis using DMT 3.0 from Affymetrix, a T-test analysis (p-value < 0.05) and a ranking analysis with regulated transcripts (FC ≥1.5/FC≤ -1.5), 84 transcripts were found to be differentially expressed coding for components involved in angiogenesis and hemostasis.

### Repression of VEGF-signaling induced angiogenesis

The vascular endothelial growth factor (VEGF) is one of the main factors involved in neo-vascularization in tumor tissues. In advanced stage of disease a negative regulation of this specific pathway (Tab.1) was observed. Specifically, the expression of VEGF itself (VEGFa and VEGFc) as were several VEGF inducers including nitric oxide (NO), platelet derived growth factor A and B polypeptide (Pdgfa, Pdgfb), fibroblast growth factor 7 (Fgf7), fibroblast growth factor binding protein 1 (Fgfbp1), fibroblast growth factor receptor 4 and 3 (Fgfr4, Fgfr3) and insulin-like growth factor binding protein 2, 3, 5 and 6 (Igfbp2, Igfbp3, Igfbp5, Igfbp6) were repressed in lung tumor tissues.

Likewise, several VEGF tyrosine kinase receptors (Flt-1, Kdr, Tek, Tie-1, and Figf) were also significantly repressed in lung adenocarcinoma. The Kdr is a VEGF specific tyrosine Kinase receptor reported as the key endothelial cell specific factor required for pathological angiogenesis^{12,13}.
Ligands for receptor tyrosine kinases (RTKs) have emerged as critical mediators of angiogenesis. Three families of ligands, vascular endothelial growth factors, angiopoietins, and ephrins, act via RTKs expressed in endothelial cells inducing vascular remodeling and angiogenesis in both embryogenesis and tumor neo-vascularization.

Angiopoietin (Agpt) binds to Tek to maintain and stabilize mature vessels whereas Agpt2 competitively binds to Tek and antagonizes the stabilizing action of Agpt, which results in destabilization of vessels^{14,15}. One important finding in lung adenocarcinoma was the disruption of the balance in the mechanism that control vessel regression and vessel growth mediated by VEGF through repression of angiopoietins including Agpt and Agpt2

Moreover, repression of ephrin ligands (Efnb2 and Efna1) and induction of ephrin receptors (Epha2 and Ephb4) was a hallmark in lung tumors. EphA2 overexpression is associated with higher tumor grade and aggressive lung cancer behavior ¹⁶ and therefore constitute an important therapeutic target in lung adenocarcinomas¹⁷.

The signaling pathways that mediate angiogenesis during tumor progression are more complex than once believed. In lung adenocarcinoma it is now demonstrated that inhibition of a specific signal transduction pathway will not be sufficient to inhibit angiogenesis. In addition, effective inhibition of VEGF angiogenic pathway may lead to selection of cells whose angiogenic activity is mediated through alternative pathways.

### Alternative pathway of angiogenesis

To verify the turn-switch of angiogenesis and to take a closer look at the expression profiles of VEGF and HGF-signaling in lung cancer cells, different stages of adenocarcinomas (1, 4, 5, 7 and 11 month- adenocarcinoma lung) were analysed by genechips.

### Relationship between HGF/SF, HGFA, MET and SPINT-1

Transcripts coding for MET oncogene (MET), the activator of HGF/SF (HGFA) and a serine protease inhibitor (SPINT-1) were identified to be regulated in lung tumors.
Hepatocyte growth factor activator **(HGFA)** is a coagulation factor Xil-like serine protease which was aberrant expressed in lung adenocarcinoma and was absent in healthy lung. This activator converts single-chains of hepatocyte growth factor (HGF/SF) to the active two-chain form and this activation is a critical limiting step in the HGF-induced signaling pathway mediated by MET-oncogene receptor tyrosine kinase¹⁸.
HGFA was increased in 4 week old- lung tumor. Re-increased levels of HGFA was identified in 5 month- lung tumors. The induction of this activator correlate well with tumor growth and tumor invasion, displaying highest level of expression at late stage of lung adenocarcinomas. Moreover, MET oncogene (MET) and a serine protease inhibitor, Kunitz type 1 (SPINT1) were also induced in lung tumors (Tab. 2a).

In contrast, an unchanged minimal expression of HGF/SF was observed by all studied lung tumor stages and lung controls (Tab. 2a). It is therefore proposed that SPINT1 is able to repress the expression and the biological function of HGF/SF in lung adenocarcinomas. Furthermore, the strongly induction of HGFA and the biological function of this activator in lung cancer remains unclear.

SPINT1 plays an important regulatory role in the pericellular activation of HGFA. It specifically, acts as a specific inhibitor of HGF/SF and as a reservoir or acceptor of HGFA on the cell surface^{19,20}. Furthermore, the balance between HGFA and SPINT-1 plays an important role in the regulation of HGF/SF activity in lung cancer. According to the inventive work it is seen that the induction of HGFA, MET and SPINT-1 in absence of HGF/SF is associated with the invasive nature of lung tumors and simultaneously, with changes on the regulation of the HGF-signaling pathway.

### Activation of MET in lung adenocarcinoma

Western blotting analysis confirmed induction of MET, HGFA and Kng1 in lung tumors. *In vivo,* Met is expressed in epithelial cells of many organs during embryogenesis and in adulthood²¹ and its activation has a crucial role in the process of epithelial-mesenchymal transition that takes place during acute injury repair²¹.

Under physiological conditions MET is transient activated in a paracrine manner, in contrast, during tumorigenesis MET is constitutively activated by different molecular alterations and either HGF-dependent or independent manner⁷. In lung adenocarcinoma the activation of MET occurs by a HGF-independent manner. The existence of cross-talk between MET and different membrane receptors suggest a role of MET in a complex and interacting networks.

The first network of interaction involved MET and an adhesive receptor CD44, which was induced in lung adenocarcinomas as well. This receptor is a cell surface glycoprotein involved in cell/cell and cell/matrix interactions and is implicated in tumor progression. Studies demonstrate that CD44 can promote MET activation and this complex participates in several signaling pathways playing an important role in tumorigenesis^{22,23}, particularly in signal transfer to the cortical actin cytoskeleton²².
Likewise, it has been reported, that in activated ras/raf/MAPK/ERK cascade MET can selectively associate with integrins²⁴. In lung adenocarcinomas integrin alpha 10 (Itgax) and integrin beta 2 (Itgb2) was identified to be induced. In this case the activity of integrin is independent from its adhesive role, because it forms an additional signaling platform necessary for the complete promotion of MET-induced invasive growth²⁴. Moreover, it has been shown that integrins are critical mediators and regulators of vascular homeostasis and physiological and pathological angiogenesis. The physical interaction of integrins with MET may promote cell adhesion and migration. It has been reported that integrin-inhibitors suppress angiogenesis and tumor progression in various animal models and are currently evaluated in clinical trials for efficacy in anti-angiogenic cancer therapies²⁵.

Interestingly, all of this receptors are individually believed to be involved in cancer progression but MET particularly, participate in the crossing of many roads leading to tumorigenesis. A constitutively activated MET receptor able to associate with membrane receptors molecules in absence of HGF/SF was identified and MET is now proposed as a potential candidate for therapies in lung adenocarcinomas.

### Kininogen 1 enhance angiogenesis in lung adenocarcinomas

High molecular weight kininogen (HMWK) is a multi-domain plasma protein that circulates in plasma primarily in its single chain form. Proteolytic cleavage of kininogen (Kng1) by plasma kallikrein releases bradykinin, and converts single chain Kininogen into active two-chain Kininogen²⁶.

Kng1 is a participant in contact phase activation of the intrinsic blood coagulation that forms a substrate on which blood factor XI is held in proximity to factor XII allowing reciprocal activation of the two factors²⁶. Kng1 was the most induced transcript in lung tumor tissues and was absent in non-trnsgenic lung. Recent studies demonstrated that kinin generated from the tissue kallikrein-kinin system enhances angiogenesis in chronic and proliferative granuloma and in the stroma surrounding a tumor and that the development of angiogenesis was significantly suppressed in kininogen-deficient rats²⁷. Furthermore, inhibition of angiogenesis by C11C1, an antibody blocking the action of proangiogenic Kng1, were observed by human fibrosarcoma on the chicken chorioallantonic membrane (CAM) assay²⁸. Functional assays measuring the effect of Kng1 in mice whole blood revealed activation of the intrinsic coagulation system by shortened activated partial thromboplastin time (aPTT) in subjects suffering from adenocarcinoma.

Kng1 is proangiogenic by releasing bradykinin. Inhibitors directed to Kng1 can serve as antiangiogenic agents with a potential for inhibiting tumor angiogenesis. Moreover, agents for the kinin-generating system and/or kinin receptor signaling may become useful tools for controlling angiogenesis in lung cancer.

### Links between hemostasis and angiogenesis

Thrombosis is a particularly common complication in human tumors^{10,29-30}. Tumoral cells express procoagulant molecules on their surface, causing activation of the coagulation cascade and stimulate tumor growth, angiogenesis and metastasis. Thus, persistent activation of the coagulation system sensitizes the host to the development of neoplasia, and individuals with idiopatic venous thromboembolism are at higher risk for developing cancer^{10,30}. A recent study reported a mouse model based on genetic manipulation of somatic cells. Targeting MET oncogene to adult liver caused slowly progressing hepatocarcinogenesis. This was preceded and accompanied by blood hypercoagulation and then evolving towards fatal internal hemorrhages¹⁰.

In lung adenocarcinoma an hypercoagulable stage by activation of the extrinsic coagulation pathway was identified. This is supported by induction of factor VII (F7) and repression of TFPI (tissue factor pathway inhibitor), an inhibitor of the extrinsic coagulation system (Tab. 3). F7 was increased in all studied lung tumors and displays highest levels at late stage of lung adenocarcinomas. Induction of FVII during tumor origin provides evidence that hemostatic disorders occur early in tumorigenesis probably as a result of tissue injury. Activation of the extrinsic coagulation pathway has been confirmed by shortened prothrombin time (PT) in blood of subjects suffering from adenocarcinoma.
Furthermore, the plasminogen activator inhibitor type 1, member 2 (serpine2 or PAI-1) and prostaglandin-endoperoxide synthase (Ptgs1 or COX1) but not COX2 were increased in lung tumors (Tab. 3). This data coincide with recent studies demonstrating that metabolites of COX-2 such prostaglandin E2 (Pge2) correlate with VEGF-signaling during tumor development³¹ and that selective overexpression of COX-1 was identified in human and mouse epithelial ovarian cancer³². Levels of downstream products of COX-1 (thromboxane B2) was found increased in urine samples of subjects suffering from lung adenocarcinoma.
At late stage of disease dysfunction of platelet adhesion was observed. Particularly, the exposition of collagen to platelets is affected by repression of thrombin receptor (F2r) and von Willebrand factor (VWF).
Adhesion of platelets results in their activation and the release of positive and negative regulators of angiogenesis. Specifically, VEGFa and VEGFc are selective products of platelet degranulation and were repressed in lung tumors. To verify the function of platelets, a PFA-100^{®} test has been performed with citrated whole blood of lung adencarcinoma- and healthy subjects. Furthermore, hematological analysis was performed. Both parameters of the PFA-100^{®} test, CEPI and CADP were significantly elevated (> 228 and > 300 sec. respectively) in whole blood of subjects suffering from lung adenocarcinoma. This data revealed a significantly abnormal platelet adhesion and aggregation in subjects suffering from adenocarcinoma as a consequence of repressed VWF in lung tumors.
Moreover, induction of Kng1, HGFA and MET are also regulated by the hypercoagulable state in lung tumors. It has been shown that thrombin promotes the activation of pro-HGFA by cleavage following Arg⁴⁰⁷ in the presence of a negatively charged substance³³. Furthermore, since blood coagulation is often linked to tissue injury, it is reasonable to postulate that plasma pro-HGFA is first activated in local injured tissue by thrombin.
With respect to thrombin, thrombomodulin (Thbd), a membrane-bound glycoprotein that forms a complex thrombin-thrombomodulin was significantly repressed, providing evidence that the action of thrombin as an anticoagulant is inhibited in subjects suffering from adenocarcinoma.
Acording to the inventive work inhibition of the fibrinolytic pathway by repression of plasminogen activator tissue (Plat), Tetranectin or plasminogen binding protein (Tna) and induction of plasminogen activator inhibitor 1 (PAI-1 or serpine2), an specific inhibitor of plasmin (Tab. 3) is reported. Notably, tetranectin was one of the most repressed genes involved in alteration of the fibrinolytic pathway in lung tumors. It is seen that tetranectin plays a role in the pathophysiology of lung adenocarcinoma and can act as a specific fibrinolytic marker in the evaluation of disease activity.

The group of therapeutical targets identified in adenocarcinomas of the lung by using the inventive method and their function for implementing the invention in its different embodiments shall be subsequently explained in closer detail:
**HGFA** is a coagulation factor XII-like serine protease which was aberrant expressed in lung adenocarcinoma and was absent in lung controls. The induction of this activator correlate well with tumor growth and tumor invasion, displaying highest level of expression at late stage of lung adenocarcinomas. The balance between HGFA and its inhibitor SPINT-1 can play an important role in the biological function of HGF/SF in lung adenocarcinomas. It is seen that the induction of HGFA, MET and SPINT-1 in absence of HGF/SF are associated with the invasive nature of lung tumors and simultaneously, with changes on the regulation and activation of the HGF-signaling pathway. HGFA is shown to have other roles during tumorigenesis but its biological function in tumoral processes remains unclear.
**Semaphorins**-a family of secreted, membrane-bound, and transmembrane proteins-play an important role in angiogenesis, tumorigenesis, and the immunological response. Semaphorins are pro-angiogenic molecules that inhibit the VEGF-pathway during angiogenesis and this effect is mediated by plexins. Moreover, the biologic effects required coupling and activation of the Met tyrosine kinase, a receptor capable to interact with several other cell surface receptors, providing it with potentially an even broader sphere of influence. In lung adenocarcinoma semaphorin 4A (Sema4A); plexin b2 (plxnb2) and Met tyrosine kinase (Met) were identified to be increased. Sema4A and Plxnb2 promote angiogenesis by association with Met and this complex constitute a novel angiogenic pathway in lung cancer.

Furthermore, **Kng1** is an initiator molecule participating in contact phase activation of the intrinsic blood coagulation cascade that forms a substrate on which blood factor XI is held in proximity to factor XII allowing reciprocal activation of the two factors. It is also involved in the activation of prekallikrein. Kng1 was the most induced transcript in lung tumor tissues and was identified as absent in healthy lung tissues. Studies demonstrated that endogenous kinin generated from the tissue kallikrein-kinin system enhances angiogenesis in chronic and proliferative granuloma and in the stroma surrounding a tumor and that the development of angiogenesis was significantly suppressed in kininogen-deficient rats. Kng1 is seen as a potent proangiogenic molecule in lung cancer and the development of an inhibitor direct to Kng1 can serve as an antiangiogenic agent with a potential for inhibiting tumor angiogenesis and other angiogenesis-mediated disorders.

Furthermore, **FVII** is seen as a principal regulator of oncogenic neovascularization and controls therefore the cancerous process.
Studies performed with inhibitors of the TF/FVlla complex shown that this complex support and promote tumor growth. Interestingly, inhibitors specific for FXa but not for FVII did not significantly inhibit primary or metastasic tumor growth. This data provides evidence that FVII can have a novel proangiogenic activity that is independent of its role of initiating coagulation and may function as a proangiogenic mechanism in lung cancer. Targeting FVII may prove efficacious in cancer treatment due to their ability to reduce the characteristic hypercoagulability of cancer and alter the fundamental biology of cancer.

The maintenance of vascular integrity and control of blood loos are regulated by a complex and coordinated system of circulating and cell-associated hemostatic factors involved in coagulation, fibrinolysis and platelet activation. However, each cascade is regulated by initiators, cofactors, feedback reactions, and inhibitors. In fact, pathways driven from cell adhesion molecules, extracellular matrix, growth factors, and other endogenous systems participate in the modulation of angiogenesis. Because hemostatic disorders occur early in tumor development as a result of tissue responses to injury, regulatory steps linking hemostasis and angiogenic pathways are seen during tumorigenesis in lung cancer.

Thrombosis is a particulary common complication in human tumors. Tumoral cells express tissue factor and other procoagulant molecules on their surface, causing activation of the coagulation cascade and stimulate tumor growth, angiogenesis and metastasis. Thus, persistent activation of the coagulation system sensitizes the host to the development of neoplasia, and individuals with idiopatic venous thromboembolism are at higher risk for developing cancer.

Moreover, it has been reported thromboembolisms and tumor hemorrhages in patients with stage IIIb/IV non-small-cell lung cancer receiving anti-angiogenic therapies, particularly those targeted against VEGF.

In order to detect patients with a risk to develop venous thromboembolism and bleeding, several hemostatic molecules are seen that help for the diagnosis and better selection of an adequate anti-angiogenic therapy in lung cancer.

The induction of a serine (cysteine) proteinase inhibitor, Clade E, member 2 (serpine2 or PAI-1) and factor VII of the coagulation (F7) with a repression of tetranectin (tna) and tissue factor pathway inhibitor (TFPI) constitute a hallmark of hemostatic disorders indicating a significant inhibition of fibrinolysis and activation of the extrinsic coagulation pathway during tumorigenesis. Moreover, repression or deficiencies of plasma von Willebrand factor (VWF), An indispensable factor for platelet adhesion was an important finding in this study that explain hemorrhages in lung cancer at late stage of the tumor.

Finally, repressed thrombomodulin (Thbd) indicate inhibition of the most important anticoagulant mechanism in hemostasis and indicate that the action of thrombin as an anticoagulant is inhibited during tumor development in lung cancer.

These data provide a possible value of these tests as tumor-markers and in order to predict thrombohemorrhagic accidents in patients with cancer.

**VWF:** Von Willebrand's disease (VWD) is now recognized to be most common inherited bleeding disorder. It arises from defects or deficiencies in a protein called von Willebrand factor (VWF). This large protein is required for normal platelet adhesion. It is a carrier protein for Factor VIII, a key protein in the coagulation cascade to the blood. Thus, VWF functions in both primary (involving platelet adhesion) by binding on platelets to its specific receptor glycoprotein lb and acts as an adhesive bridge between the platelets and damaged subendothelium at the site of vascular injury. In secondary (involving FVIII) hemostasis, VWF protects FVIII from degradation and delivers it to the site of injury.

The correct diagnosis and sub-classification of a patient's VWD is crucial because the presenting biological activity of VWF determines the hemorrhagic risk, and since subsequent clinical management will differ accordingly.
The repression of VWF at late stage of lung adenocarcinoma and altered platelets adhesion was an important finding that not only explain hemorrhages in lung cancer at late stage of the tumor but also the repression of several growth factors incluging VEGFa and VEGFc.

**FVII** and **TFPI**: In lung adenocarcinoma an hypercoagulable stage by activation of the extrinsic coagulation pathway was identified. This is supported by induction of factor VII (F7) and repression of TFPI (tissue factor pathway inhibitor), an inhibitor of the extrinsic coagulation system. F7 was increased in all studied lung tumors and display highest levels at late stage of lung adenocarcinomas. Induction of FVII during tumor origin provides evidence that hemostatic disorders occur early in tumorigenesis and may regulate differents angiogenic pathways.

**Tna, Plat and serpine2:** Inhibition of the fibrinolytic pathway by repression of plasminogen activator tissue (Plat), Tetranectin or plasminogen binding protein (Tna) and induction of plasminogen activator inhibitor 1 (PAI-1 or serpine2), an specific inhibitor of plasmin, is reported according to the invention. Notably tetranectin was one of the most repressed gene involved in alteration of the fibrinolytic pathway in lung tumors (identified as absent in lung tumor tissues). Tetranectin is seen to play a role in the phatophysiology of lung adenocarcinoma and can act as a specific fibrinolytic marker in the evaluation of disease activity. Recent studies suggest that tetranectin is may involved in fibrinolysis and proteolysis during tissue remodeling, but its precise biological function remains unknown.

**Thrombomodulin:** Coagulation is also controlled by an anticoagulant pathway composed of thrombin/thrombomodulin complex and activation of protein C. Protein C inhibits coagulation by inactivation of essential cofactos required for thrombin formation, factors Va and VIIIa.
Thrombomodulin (Thbd), a membrane-bound glycoprotein, has a high affinity of binding to thrombin and converts thrombin from a procoagulant to an anticoagulant molecule. Thbd was significantly repressed, providing evidence of the action of thrombin as an anticoagulant is inhibited in lung adenocarcinomas.

Over the past several years, various compounds have been developed to inhibit secreted proteins such as the much studied VEGF-signaling, which clearly plays a role in tumor angiogenesis. To date, though, none of this compounds have demonstrated convincing efficacy in human therapies. Moreover, thromboembolisms and tumor hemorrhages in patients with stage IIIb/IV non-small-cell lung cancer receiving anti-angiogenic therapies, particularly those targeted against VEGF were reported³⁵.
In lung adenocarcinomas, proangiogenic molecules were identified to be absent in normal lung tissues and strongly induced in tumor cells. Kng1 exhibits angiogenic activities by liberating bradykinin. Studies have demonstrated that kng1 monoclonal antibodies can inhibit angiogenesis in the CAM assay, human colon carcinoma growing as a xenograft in nude mice, and murine hybridomas growing in syngeneic hosts²⁸. Inhibitors for Kng1 can now serve as an anti-angiogenic agents with a potential for inhibiting tumor angiogenesis in cancer therapies.

Moreover, studies performed with inhibitors of the TF/FVlla complex show that this complex supports and promotes tumor growth. Interestingly, specific inhibitors for FXa but not for FVII did not significantly inhibit primary or metastasic tumor growth. This data provide evidence that FVII can have a novel proangiogenic activity that is independent of its role of initiating coagulation and may function as a proangiogenic mechanism in lung cancer. Targeting FVII may prove efficacious in cancer treatment due to its ability to reduce the characteristic hypercoagulability of cancer and therefore alter the fundamental biology of cancer.

Induction of HGFA, SPINT-1 and MET by a HGF-independent mechanism may help to understand the molecular mechanism of HGF-signaling during tumorigenesis in lung cancer. Moreover, targeting the receptor MET but not its ligand HGF/SF is an effective way to interfere with many pathways participating in tumor progression and metastasis.

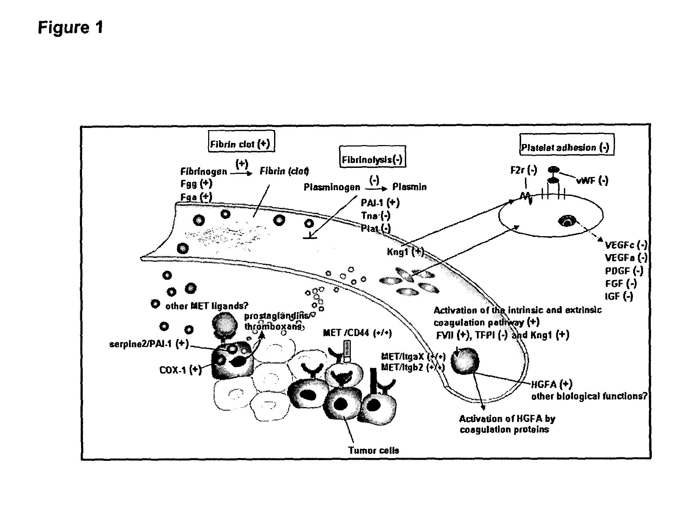

Because hemostatic disorders occur early in tumor development probably as a result of tissue responses to injury, regulatory steps linking hemostasis and angiogenic pathways (Fig. 1) are postulated.

Contribution of platelets to hemostasis and angiogenesis is mediated either directly by thrombin through its receptor F2r or indirect by binding of platelets to collagen through VWF. The repression of VWF at late stage of disease and altered platelet adhesion was an important finding in this study that not only explain hemorrhages in lung cancer at late stage of the tumor but also the repression of growth factors including VEGFa and VEGFc. This has to be carefully considered when selecting suitable targets for cancer therapy where VEGF is regarded as a potential angiogenic candidate. Moreover, it has been shown that kininogens are able to inhibit the thrombin-induced aggregation of platelets and this modulation appears to be through the GP Ib-IX-V receptor³⁶. The inhibitory effect of Kininogen in thrombin-induced platelet aggregation was observed in situations such reocclusion after angioplasty or thrombolysis³⁷ without interfering with the other hemostatic functions of thrombin and its action on fibrinogen to form a fibrin clot.

In order to detect patients with a risk to develop venous thromboembolism and bleeding, hemostatic molecules are now identified that can help for the diagnosis and better selection of an adequate anti-angiogenic therapy in lung cancer.

Induction of serpine2 and FVII with repression of Tna and TFPI are important hemostatic parameters indicating inhibition of fibrinolysis and activation of the extrinsic coagulation pathway. Moreover, plasma von Willebrand factor (VWF) has been identified as an indispensable factor for platelet adhesion, deficiencies of VWF are associated with bleeding at late stage of the tumor. Repressed thrombomodulin indicates inhibition of the most important anticoagulant mechanism in hemostasis. These data provide a value of these tests as tumor-markers and in order to predict thrombohemorrhagic accidents.

Experiments were performed using, inter alia, the following methods and means.

**Extraction of total RNA.** Normal lung tissues (n=6) and tumoral lung tissues (n=8) were used for RNA preparations.RNA extractions and purifications were performed with RNeasy midi kit (Qiagen, Cat.N:75144) according to the manufacturer's instructions.

**cDNA synthesis.** First-strand cDNA was synthesized from 10 µg of total RNA with a oligo(dT)₂₄ primer (PROLIGO Primers and Probes; SuperScript II RNase H- Reverse Transkriptase, 5x First-Strand Buffer und 0,1 M DTT (Invitrogen;18064-014 oder 18064-071), dNTP Mix, 10mM (Invitrogen; 18427-013). Second-strand synthesis was synthesized in 20 µl of first-strand reaction mix at 42°C for 1 h using 5xSecond-Strand Buffer (Invitrogen; 10812-014), DNA Ligase E.coli, 10U/µl (Invitrogen; 18052-019), DNA Polymerase I E.coli, 10U/µl (Invitrogen; 18010-025), RNase H; 2U/µl (Invitrogen; 18021-14;18021-071), T4-DNA Polymerase; 5U/µl (Invitrogen; 18005-025), EDTA Disodium Salt, 0,5M solution (SIGMA; P/N E7889). Purification of cDNA was performed using the GeneChip^{®} Sample Cleanup module according to the Affymetrix protocol.

**In vitro transcription reaction.** After second-strand synthesis, biotin-labeled cRNA was generated from the cDNA sample by an in vitro transcription reaction with the BioArray RNA transcript labeling kit (Enzo Diagnostics, Farmingdale, N.Y.) with biotin-labeled CTP and UTP. The labeled cRNA was purified with RNeasy spin columns (Qiagen). 15 µg of each cRNA sample was fragmented at 94°C for 35 min in fragmentation buffer (40 mM Tris-acetate, pH 8.1, 100 mM potassium acetate, and 30 mM magnesium acetate) and then used to prepare 300 µl of the hybridization mix. A biotinylated oligonucleotide, B2, that hybridizes to unique features at the center and four corners of each chip was used to orient the probe sets on the chip.

**Microarray Hybridization.** Affymetrix GeneChips were placed into the GeneChip Hybridisation Oven 640 (Affymetrix). Hybridizations proceeds at 45°C for 16 hours at 60 rpm. The arrays were removed from the chamber. Washed, dried and dyeing were performed in the wash Station 400 (Affymetrix) according to the Affymetrix array protocol.

**Microarray Scanning and Data Acquisition.** The hybridized arrays were scanned with a calibrated GeneArray Scanner (Agilent) at wavelengths 570 nanometers (Pixel 3 µm). Each array was 3 x scanned. The software used for data acquisition was the GeneChip orerating software GCOS (Affymetrix).

**Statistical analysis.** Array data was normalized using scaling or per-chip normalization to adjust the total or average intensity of each array to be approximately the same. The scale factor was according to the Affymetrix recommended setting and used to generate a microarray quality control and data report. Features that exhibit differential expression greater than 1.5-fold were deemed significant and reported in an Excel spreadsheet with gene annotation and ontological data derived from the NetAffx Analysis center. A t-test and ranking analysis was performed using Data Mining Tool (DMT) 3.0 from Affymetrix. A stringent comparison between normal and tumoral tissues was performed according to the consistency of gene expression changes. Only genes that exhibit a 100% concordance in the change direction with a p-value < 0.05 (T-test) and a FC ≥1.5/FC≤-1.5 were reported.

**RT=PCR.** Specific amplimers for each gene were obtained from invitrogen life technologies. Sequences are given for the forward and reverse amplimers respectively.
VEGFa: ACATCTTCAAG-CCGTCCTGT; GCGAGTCTGTGTTTTTGCAG. VEGFc:
TGTGTCCAGCGTAGATGAGC; TGAGG-TAACCTGTGCTGGTG: kdr:
CTTCCTGACCTTGGAGCATC; CAGAGCAACACACCGAAAGA. SPINT-1:TAGCAATGGCTGCTGTATCG; CCGAAGACCAAACACATCCT. F7:
GGAACAGTGCTCC-TTTGAGG; TTTGCAGGACACCTCATCTG. Kng-1:
GAGGTGCTTGGTCATTCCAT; CTCCGGAAA-GGAGAAAAACC. HGF:
AGAGGTCCCATGGATCACAC; GACAGGGAATTCCATTCCAA. Met:
TGTCAGCATCGCTCAAATTC; GTGGAGACTCTCTCGCAGCTCT: HGFA:
GCTTCCTGGGAAAT-GGTACA; CCTCTTGCCACAGGTAGGAC. FIt1:
CCCTGATGGGCAAAGAATAA; TCCGCTGCC-TTATAGATGCT.
RT-PCR:20 ng cDNA were analysed in 20 µl reactions (94°C, 1 min.; 55-58°C, 1 min.; 72°C, 2 min.) PCR-reactions were performed using ABgene Thermo- Start Taq (Cat-Nr.:AB-1908/B),10x Reaction Buffer (15 mM MgCl₂), and 10 mM dNTP from MBI Fermentas (Cat-Nr.: R0181). PCR-reactions were done for 25-40 cycles to determine the linear range of amplification for each primer set. Quantification of the bands was performed with the Kodak 1 D 3.5 Network software.

**Western blot.** 100 µg proteins were separated using a 12% gradient SDS-PAGE gel and transferred to a PVDF membrane (NEN, Cat-Nr: NEF1002) at 40 V for 1 h. The membrane was blocked with 1 xRoti-Block (Roth, Cat-Nr: A151.1) in 1 x TBS-Buffer over night at 4°C. Antibodies were obtained from Santa Cruz Biotechnology. HGFA-L (N-19) sc-1371; MET (SP260) sc-162; VEGF (C-1) sc-7269 and Kininogen LC (C11C1) sc-23915. Secondary antibody mouse/rabbit IgG (Chemicon, Cat-Nr: AP 160P and AP132P) were used at 1:5000 dilution. Detection of proteins were performed using a chemiluminiscence reagent (NEN, Cat-Nr: NEN 104) and exposed using a Kodak IS 440CF software.

### Histopathology

**aPTT and PT.** The quantitative in vitro determination of the aPTT test and the modified prothrombin time (PT, Quick value) were performed with citrated whole blood. Reagents were obtained from Diagnostica Stago (Roche), Cat-Nr: 0126535 (PT) and 0126551 (aPTT). Assays were performed according to the manufacturer's instructions.

PFA100. Citrated whole blood (non-centrifuged) within four hours of collection was aspirated under high shear, through a capillary tube onto a collagen/ADP (CADP) coated membrane, or a coliagen/epinephrine (CEPI) membrane containing a 150 µm diameter central aperture. Platelets aggregation eventually produces total closure of the aperture and the time until this occurs is measured.

**Hematology.** hematologic profiles were performed using the kx-21 N (Sysmex). Diluted EDTA/ whole blood (1:2) were used for the determination of erythrocytes and reticulocytes (RBC), white blood cells (WBC), platelet counts (PLT), hematocrit (HCT). Hemoglobin (HGB) was measured at 540 nm according to the Sodium-lauryl method (SLS-HB-method).

The characteristics of the invention being disclosed in the preceeding description, the subsequent tables, figures, and claims can be of importance both singularly and in arbitrary combination for the implementation of the invention in its different embodiments.

### Literature

1. Hanahan, D., Christofori, G., Naik, P., Arbeit, J. Transgenic mouse models of tumor angiogenesis: the angiogenic switch, its molecular controls, and prospects for preclinical therapeutic models. Eur J Cancer 32A, 2386-93 (1996).
2. Dvorak, H.F., Brown, L.F., Detmar, M., Dvorak, A.M. Vascular permeability factor/vascular endothelial growth factor, microvascular hyperpermeability and angiogenesis. Am J Pathol 146, 1029-39 (1995).
3. Ribatti, D., Vacca, A., Presta, M. The discovery of angiogenic factors: a historical review. Gen Pharmacol. 35, 227-231 (2000).
4. Ferrara, N. Role of vascular endothelial growth factor in regulation of physiological angiogenesis. Am J Physiol Cell Physiol. 280, 1358-66 (2001).
5. Bussolino, F. et al. Hepatocyte growth factor is a potent angiogenic factor which stimulates endothelial cell motility and growth. J Cell Biol. 119, 629-641 (1992).
6. Parr, C.; Jiang, W.G. Expression of hepatocyte growth factor/scatter factor, its activator, inhibitors and the c-Met receptor in human cancer cells. Int J Oncol. 19, 857-63 (2001).
7. Corso, S. et al. Cancer therapy: can the challenge be MET?. Trends Mol Med. 11, 284-92 (2005).
8. Xin, X. et al. Hepatocyte growth factor enhances vascular endothelial growth factor-induced angiogenesis in vitro and in vivo. Am J Pathol. 158,1111-1120 (2001).
9. Gerritsen, M.E. et al. Using gene expression profiling to identify the molecular basis of the synergistic actions of hepatocyte growth factor and vascular endothelial growth factor in human endothelial cells. Br J Pharmacol. 140, 595-610 (2003).
10. Boccaccio, C. et al. The MET oncogene drives a genetic programme linking cancer to haemostasis. Nature 434, 396-400 (2005).
11. Bajzar, L. Thrombin activatable fibrinolysis inhibitor and an antifibrinolytic pathway. Arterioscler Thromb Vasc Biol. 20, 2511-8 (2000).
12. McMahon, G. VEGF Receptors Signaling in tumor Angiogenesis. Oncologist. 1, 3-10 (2000). Review.
13. Takeda, N. et al. Endothelial PAS domain protein 1 gene promotes angiogenesis through the transactivation of both vascular endothelial growth factor and its receptor, Flt-1. Circ Res. 95,146-53 (2004).
14. Maisonpierre, P. C. et al. Angiopoietin-2, a natural antagonist for Tie 2 that disrupts in vivo angiogenesis. Science 277, 55-60 (1997).
15. Sato, T. N. et al. Distinct roles of the receptor tyrosine kinases Tie-1 and Tie-2 in blood vessel formation. Nature 376, 70-74 (1995).
16. Kinch, M.S. et al. Predictive value of the EphA2 receptor tyrosine kinase in lung cancer recurrence and survival. Clin Cancer Res. 9,613-8 (2003).
17. Brantley-Sieders et al. Impaired tumor microenvironment in EphA2-deficient mice inhibits tumor angiogenesis. FASEB J. 19, 1884-1886 (2005).
18. Miyazawa, K., Shimomura, T., Kitamura, N. Activation of hepatocyte growth factor in the injured tissues is mediated by hepatocyte growth factor activator. J Biol Chem. 271, 3615-8 (1996).
19. Kataoka, H. et al. Pericellular activation of hepatocyte growth factor/scatter factor (HGF/SF) in colorectal carcinomas: roles of HGF activator (HGFA) and HGFA inhibitor type 1 (HAI-1). Hum Cell 14, 83-93 (2001).
20. Nagashima, M. et al. Hepatocyte growth factor (HGF), HGF activator, and c-Met in synovial tissues in rheumatoid arthritis and osteoarthritis. J. Rheumotol. 28, 1772-8 (2001).
21. Birchmeier, C.; Gherardi E. Developmental roles of HGF/SF and its receptor, the c-Met tyrosine kinase. Trends Cell Biol. 8, 404-10 (1998).
22. Orian-Rousseau, V. et al. CD44 is required for two consecutive steps in HGF/c-Met signaling. Genes Dev. 16, 3074-86 (2002).
23. Wielenga, V.J. et al. Expression of c-Met and Heparan-Sulfate Proteoglycan Forms of CD44 in Colorectal Cancer. Am. J. of pathol. 157, 1563-73 (2000).
24. Trusolino, L., Berotti, A., Comoglio, P.M. A signaling adapter function for alpha6beta4 integrin in the control of HGF-dependent invasive growth. Cell 107, 643-54 (2001).
25. Abdollahi, A. et al. Inhibition of alpha(v)beta3 integrin survival signaling enhances antiangiogenic and antitumor effects of radiotherapy. Clin Cancer Res. 11, 6270-9 (2005).
26. Herwald, H., Mörgelin, M., Svensson and Sjöbring, U. Zinc-dependent conformational changes in domain D5 of high molecular mass kininogen modulate contact activation. Eur. J. Biochem. 268, 396-404 (2001).
27. Hayashi, I. et al. Supressed angiogenesis in kininogen-deficiencies. Lab Invest. 82, 871-80 (2002).
28. Colman, R.W. et al. Inhibition of angiogenesis by antibody blocking the action of proangiogenic high-molecular-weight-kininogen. J Thromb Haemost. 1, 164-70 (2003).
29. Lip, G.Y., Chin, B.S., Blann, A.D. Cancer and the prothrombotic state. Lancet Oncol 3, 27-34 (2002).
30. Stenina, O.I., Plow, E.F. MET orchestrates cancer and blood coagulation. Nature Medicine 11, 376-377 (2005).
31. Bradbury, D. et al. Vascular endothelial growth factor induction by prostaglandin E2 in human airway smooth muscle cells is mediated by E prostanoid EP2/EP4 receptors and SP-1 transcription factor binding sites. J Biol Chem. 280, 29993-30000 (2005).
32. Daikoku, T. et al. Cyclooxygenase-1 is overexpressed in Multiple Genetically Engineered Mouse Models of epithelial ovarian cancer. Cancer Res. 66, 2527-31 (2006).
33. Shimomura, T. et al. Activation of the zymogen of hepatocyte growth factor activator by thrombin. J. Biol. Chem. 268, 22927-32 (1993).
34. Wewer, U.M. et al. A potential role for tetranectin in mineralization during osteogenesis. J Cell Biol. 127,1767-75 (1994).
35. Daly, M.E., Makris, A., Reed, M., Lewis, C.E. Hemostatic Regulators of Tumor Angiogenesis: A Source of Antiangiogenic Agents for Cancer Treatment? J. Natl. Cancer Inst. 95,1660-73 (2003).
36. Bradford, H.N. et Thrombin al. Human kininogens regulate binding to platelets through the Glycoprotein lb-IX-V Complex. Blood 90, 1508-15 (1997).
37. Puri, R.N. and Colman, R.W. Reocclusion after thrombolytic therapie: Strategies for inhibiting thrombin-induced platelets aggregation. Blood Coag Fibrinolysis 4, 465-478(1993).

### Tables

**Table 1 Genes significantly repressed ( ≤ -1.5-fold, P< 0.1) by VEGF-signalling induced angiogenesis in advanced stage of lung adenocarcinoma. (†) represent genes identified as present in normal lung tissues and absent in lung tumor tissues.**

| ***Description of gene* / *gene product*** | ***Symbol*** |
|---|---|
| vascular endothelial growth factor A | Vegfa |
| vascular endothelial growth factor C | Vegfc |
| epidermal growth factor receptor | Egfr |
| fibroblast growth factor 7 | Fgf7 |
| fibroblast growth factor receptor 4 | Fgfr4 |
| fibroblast growth factor receptor 3 | Fgfr3 |
| platelet derived growth factor, B polypeptide | Pdgfb |
| platelet derived growth factor, A polypeptide | Pdgfa |
| insulin-like growth factor binding protein 2 | lgfbp2 |
| insulin-like growth factor binding protein 3 | lgfbp3 |
| insulin-like growth factor binding protein 5 | lgfbp5 |
| insulin-like growth factor binding protein 6 | lgfbp6 |
| protease,serine 11 (Igf binding) | Prss11 |
| c-fos induced growth factor | Figf |
| FMS-like tyrosine kinase 1 | Flt1 |
| kinase insert domain protein receptor | Kdr |
| tyrosine kinase receptor 1 | Tie1 |
| endothelial-specific receptor tyrosine kinase | Tek (Tie-2) |
| angiopoietin | Agpt |
| angiopoietin-like 4 | Angptl4 |
| endothelial differentiation, G-protein-coupled | Edg6 |
| receptor 6 | |
| endothelial differentiation, sphingolipid G-protein- | Edg3 |
| coupled receptor, 3 | |
| endothelial differentiation sphingolipid G-protein- | Edg1 |
| coupled receptor 1 | |
| endothelial PAS domain protein 1 | Epas1 |
| Ephrin B2 | Efnb2 |
| Ephrin A1 | Efna 1 |

**Table 2: Angiogenic and hemostatic factors regulated in lung adenocarcinoma**

| | | |
|---|---|---|
| ***Description*** | **Gene *Symbol*** | |
| hepatocyte growth factor activator | Hgfac | induced |
| coagulation factor VII | F7 | induced |
| Kininogen 1 | Kng1 | induced |
| semaphorin 4A | Sema4A | induced |
| Plexin B 2 | Plxnb2 | induced |
| tetranectin (plasminogen binding protein | Tna | repressed |
| Thrombomodulin | Thbd | repressed |
| plasminogen activator, tissue | Plat | repressed |
| tissue factor pathway inhibitor | Tfpi | repressed |
| Von Willebrand factor homolog | Vwf | repressed |
| serine (cysteine)proteinase inhibitor,Clade A, | | |
| member 1 | Serpine2 | induced |

**Table 3 Differentially expressed genes ( ≤-1.5-fold or ≥1.5-fold, P< 0.1) related with hemostasis: fibrinolysis, coagulation and platelets in advanced stage of lung adenocarcinoma. (†) represent genes identified as present in normal lung tissues and absent in lung tumor tissues. (‡)represent genes identified as present in tumor lung tissues and absent in normal lung tissues**

| ***Description of gene* / *gene product*** | ***Gene Symbol*** | ***Fold change*** |
|---|---|---|
| tetranectin (plasminogen binding protein) | Tna | < -1.5-fold† |
| Thrombomodulin | Thbd | < -1.5-fold |
| plasminogen activator, tissue | Plat | < -1.5-fold |
| tissue factor pathway inhibitor | Tfpi | < -1.5-fold |
| serine (cysteine)proteinase inhibitor,Clade G, | serping1 | < -1.5-fold |
| member 1 | | |
| serine (cysteine)proteinase inhibitor,Clade A, | serpina1 | < -1.5-fold |
| member 1 | | |
| serine (cysteine)proteinase inhibitor,Clade E. | serpine2 | > 1.5-fold |
| member 2 | | |
| prostaglandin-endoperoxide synthase 1 | Ptgs1 | > 1.5-fold |
| Prostaglandin E synthase | Ptges | > 1.5-fold |
| coagulation factor VII | F7 | > 1.5-fold |
| kininogen 1 | Kng1 | > 1.5-fold |
| fibrinogen, gamma polypeptide | Fgg | > 1.5-fold‡ |
| fibrinogen, alpha polypeptide | Fga | > 1.5-fold‡ |
| early growth response 1 | Egr1 | > 1.5-fold |
| multiple coagulation factor deficiency 2 | Mcfd2 | > 1.5-fold |
| thrombospondin 1 | Thbs1 | > 1.5-fold |
| Coagulation factor II (thrombin) receptor | F2r | < -1.5-fold |
| Von Willebrand factor homolog | Vwf | < -1.5-fold |

### Legends to Figures

**Figure 1. Hemostatic and pro-angiogenic factors controlling angiogenesis at late stage of lung adenocarcinomas.** Adapted from Stenina, O.I., Plow, E.F. ²⁹. Induction of Kng1, HGFA and MET in lung adenocarcinomas. The constitutively activation of MET occur through an independent HGF/SF mechanism. The existence of cross-talk between MET and different membrane receptors such CD44 or integrins suggest a role of MET in a complex and interacting networks. At late stage of lung tumors activation of the extrinsic pathway of the coagulation by induction of FVII and inhibition of the fibrinolytic pathway was observed. The adhesion of platelets by exposition of collagen to platelets is affected by repression of thrombin receptor (F2r) and specifically by repression of the von Willebrand factor (VWF). This may explain the repression of VEGFc, VEGFa and hemorrhages at late stage of lung cancer. Finally, induction of serpine2 and COX-1 may support the thrombohemorrhagic phenotype in lung cancer.
The foregoing description of preferred embodiments of the invention has been presented for the purposes of illustration and description. It is not intended to be exhaustive or to limit the invention to the precise form described, and many modifications and variations are possible in light of the teaching above.
The embodiments were chosen and described in order to best explain the principles of the invention and its practical applications to thereby enable others skilled in the art to best utilize the invention in various embodiments and with various modifications as are suited to the particular use contemplated. The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A method for identifying therapeutical targets in tumors, in particular in advanced stage tumor malignancies, such as a late stage adenocarcinoma of the lung may be, comprising the steps of
- isolating RNA (1) from the tissue of the tumor;
- determining for the isolated RNA (1) a gene expression profile (2) of at least two genes, wherein at least one gene (3) is coding for a VEGF activity modulator and at least one gene (4) is coding for a hemostatic factor by screening the presence of mRNA coding for the factors to be screened and by determining the levels of expression of thereof;
- determining the changes of expression of the at least two genes screened by the gene expression profile (2) in comparison with healthy tissue or with an early stage tumor; and
- identifying the therapeutical target as a hemostatic factor, being
(a) upregulated or downregulated,
if parallely
(b) the VEGF acitivity modulator is downregulated or mainly non changed, in the gene expression profile (2) in comparison with healthy tissue or with an early stage tumor.

2. Method as claimed in claim 1, wherein
- RNA (1) is isolated from the tissue of an advanced stage tumor malignancy of the breast, colon, lung, stomach, prostate, pancreas or cervix, in particular of a human patient; and/or
- the gene (3) is coding for VEGF or for a cytokine inducing VEGF or for a VEGF tyrosine kinase receptor; and/or
- the gene (4) is coding for a hemostatic factor participating in the induction of fibrinolysis, coagulation or the formation of platelets; and/or
- a standard is used for determining the changes of expression, in particular the level of gene expression of at least one of the genes to be screened derived from a gene expression profile for a healthy tissue or for an early stage tumor of the organ, wherein the tumor is formed, is used; and/or
- the therapeutical target is identified as a hemostatic factor **characterized by** an at least 1.5 fold increase or decrease of its' expression level in comparison with healthy tissue or with an early stage tumor.

3. Method as claimed in claims 1-2, wherein
- RNA (1) is isolated from a late stage adenocarcinoma, in particular from an advanced stage tumor of the lung; and/or
- at least one of the two genes coding for a hemostatic and/or angiogenic factors are selected from the group of genes in Table 2; and/or
- the gene (3) is selected from the group of genes in Table 1; and/or
- the gene (4) is selected from the group of genes in Table 3 and/or HGFA, Sema4A, plxnb2; and/or
- the at least two genes coding for a hemostatic and/or angiogenic factors are selected from the group of genes in Table 3 and/or HGFA, cMet, Spint1, VEGFa, VEGFc, Fit1, Kdr, Figf, Tie1, Tek; and/or
- the standard comprises values for the expression levels of a VEGF activity modulator and of a hemostatic factor screened and/or values for HGF or β-Actin, in particular being determined parallely to the gene expression profile (2); and/or
- the levels of expression of the at least two genes coding for the factors screened are normalized with respect to the levels of expression of the respective genes in healthy tissue or in an early stage tumor of the organ, wherein the tumor is formed, in particular by using the standard for normalizing; and/or
- the VEGF acitivity modulator is downregulated by an at least 1.5 fold decrease of its' expression level compared with the standard.

4. Method as claimed in claims 1-3, wherein the gene expression profiling comprises the synthesis of a cDNA library (5) derived of the isolated RNA (1) and/or the synthesis a cDNA library (6) derived of RNA of the healthy tissue or of the early stage tumor of the organ, wherein the tumor is formed, by RT-PCR and/or by quantitative RT-PCR.

5. Method as claimed in claim 4, wherein the gene expression profiling further comprises the steps of
- amplifying cDNA sequences of the hemostatic and/or angiogenic factors to be screened by a PCR, such as a thermocycler PCR may be, wherein the cDNA library (5) and/or (6) is mixed with synthetic primers, having complementary sequences for specifically annealing with the cDNA copies of the hemostatic and/or angiogenic factor mRNA,
- separating the amplified cDNA sequences by gel electrophoresis of the PCR reaction mixtures,
- vizualizing the separated PCR products.

6. Method as claimed in claims 4-5, wherein, for vizualizing the separated PCR products,
- labeled synthetic primers are used in the PCR, and/or
- a substance, in particular a dye such as ethidium bromide may be, intercalating in double stranded oligonucleotides, is used for labelling the PCR products.

7. Method as claimed in claim 4, wherein the gene expression profiling further comprises the steps of
- synthesizing a cRNA library (6) derived of the cDNA library (5) and/or synthesizing a cRNA library (7) derived of the cDNA library (6) by second strand cDNA synthesis and *in vitro* transcription of the double stranded cDNA;
- producing a RNA fragment library (8) derived of the cRNA library (6) and/or producing a RNA fragment library (9) derived of the cRNA library (7) by hydrolytic cleavage into RNA fragments, in particular by metal-induced hydrolysis into RNA fragments of the length of 35-200 bases;
- performing a hybridization assay by incubating an oligonucleotide array (10) including spatially addressed solid phase bound oligonucleotide sequences coding for the at least two hemostatic and/or angiogenetic factors to be screened with a solution of dissolved cRNA fragment library (8) and/or performing a hybridization assay by incubating an oligonucleotide array (11) including spatially addressed solid phase bound oligonucleotide sequences coding for the at least two hemostatic and/or angiogenetic factors to be screened with a solution of dissolved cRNA fragment library (9);
- scanning the hybridization patterns of the oligonucleotide array (10) and/or (11).

8. Method as claimed in claim 7, wherein labelled ribonucleotides, such as biotin labelled ribonucleotides may be, are used for the *in vitro* transcription.

9. Method as claimed in claims 7-8, wherein an oligonucleotide microarray is used for performing the hybridization assay.

10. Method as claimed in claims 1-9, wherein HGFA, Sema4A, plxnb2, Kng1, FVII, VWF, TFPI, Tna, Plat, serpine2, and/or thrombomodulin is identified as the target.

11. Use of one or more therapeutical targets, in particular of targets being identified according to the method as claimed in claims 1-10, for identifying, determining, and targeting angiogenesis and hemostasis related to adenocarcinomas of the lung, such as identifying diagnostic markers in body fluids or tissue, determining the presence of mRNA or proteins in tumor cells, and targeting mRNA or proteins by therapeutical means may be, comprising the steps of
- isolating a biological sample (12) from an organism, in particular from a human patient, suffering from lung cancer or from an organism to be tested for its susceptibility to lung cancer, and
- determining the levels of HGFA, Sema4A, plxnb2, Kng1, FVII, VWF, TFPI, Tna, Plat, serpine2, and thrombomodulin or of fragments of thereof or of a selection of thereof in the biological sample (12) by screening the presence of said proteins or of fragments of thereof or of mRNA coding for the same.

12. Use as claimed in claim 11, further comprising the steps of
- isolating a biological sample (13) from a healthy organism, in particular from a human being;
- pairwisely comparing the gene expression profiles determined for the isolated biological samples (12) - (13) by correlating the levels measured.

13. Use as claimed in claims 11-12, wherein
- the isolated biological sample is a tissue, a cell, a cellular compartment, total RNA, total protein or a body fluid; and/or
- the levels of HGFA, Sema4A, plxnb2, Kng1, FVII, VWF, TFPI, Tna, Plat, serpine2, and thrombomodulin or of fragments of thereof or of a selection of thereof is determined in the biological sample (13) by screening the presence of said proteins or of fragments of thereof or of mRNA coding for the same.

14. Use as claimed in claim 11-13, wherein
- the biological sample is isolated from a lung adenocarcinoma or from the blood of the organism; and/or
- the levels of at least two factors selected from the group of HGFA, Sema4A, plxnb2, Kng1, FVII, VWF, TFPI, Tna, Plat, serpine2, and thrombomodulin or of fragments of thereof or of mRNA coding for the same or of a selection of thereof is determined in the isolated biological sample (12) and/or in the isolated biological sample (13).

15. Use as claimed in claims 11-14, , wherein
- the organism suffering from lung cancer or the organism to be tested for its susceptibility to lung cancer is a human patient or a transgenic animal, such as a c-myc mouse may be; and/or
- the levels of at least three factors selected from the group of HGFA, Sema4A, plxnb2, Kng1, FVII, VWF, TFPI, Tna, Plat, serpine2, and thrombomodulin or of fragments of thereof or of mRNA coding for the same or of a selection of thereof is determined in the isolated biological sample (12) and/or in the isolated biological sample (13).

16. Use as claimed in claims 11-15, wherein the level is determined by gene expression profiling, by western blotting or by histopathology and/or wherein the activated partial thromboplastin time **(aPTT)** and/or the prothrombin time **(PT)** and/or the capillary in vitro bleeding time (PFA100) and/or a hematologic profile is determined.

17. Use as claimed in claims 12-16, wherein the gene expression profiling comprises the steps of
- synthesizing a cDNA library derived of the isolated RNA by RT-PCR,
- synthesizing a cRNA library, derived of the cDNA library by second strand cDNA synthesis and *in vitro* transcription of the double stranded cDNA,
- producing a RNA fragment library derived of the cRNA library by hydrolytic cleavage into RNA fragments,
- performing a hybridization assay by incubating an oligonucleotide array including spatially addressed solid phase bound oligonucleotide sequences coding for the at least two oligonucleotide sequences to be screened or for parts of thereof or for sequences being complementary of the same, with the cRNA fragment library,
- scanning the hybridization pattern of the oligonucleotide array.

18. Use as claimed in claims 11-17, wherein wherein labelled ribonucleotides, such as biotin labelled ribonucleotides may be, are used for the *in vitro* transcription.

19. Use as claimed in claims 11-18, wherein oligonucleotide microarrays are used for performing the hybridization assays.

20. Use as claimed in claims 11-19, wherein antibodies directed against HGFA, Sema4A, plxnb2, Kng1, FVII, VWF, TFPI, Tna, Plat, serpine2 or thrombomodulin are used.

21. Use as claimed in claims 11-20, wherein one or more genes and/or one or more gene products of thereof selected from the group of HGFA, Sema4A, plxnb2, Kng1, FVII, VWF, TFPI, Tna, Plat, serpine2, thrombomodulin and/or their mutants and/or variations and/or parts thereof and/or derived molecules is used to screen for and to identify drugs targeting angiogenesis and hemostasis related to tumor malignancies of the lung, in particular drugs against adenocarcinoma of the lung.

22. Use as claimed in claims 11-21, wherein one or more genes selected from the group of HGFA, Sema4A, plxnb2, Kng1, FVII, VWF, TFPI, Tna, Plat, serpine2, thrombomodulin and/or their mutants and/or variations and/or parts thereof and/or related molecules and/or their gene products and/or derived structures are incubated with a compound to be tested and changes in the expression of said genes and/or derived sequences and/or the function of said gene products and/or derived structures are determined.

23. Use as claimed in claims 11-22, wherein drugs regulate the expression of one or more of said genes and/or the function of one or more of said gene products and/or their derived molecules and are used for the (production of means for) treatment of tumor malignancies of the lung, in particular of a lung adenocarcinoma.

24. Use as claimed in claims 11-23, wherein DNA and/or or related molecules encoding one or more of said gene products and/or derived structures are used.

25. Use as claimed in claims 11-24, wherein one or more polypeptides, peptides and/or derived molecules having the function of one or more of said gene products, are used.

26. Procedure for identifying, labelling and treating of tumor malignancies of the lung, such as a lung adenocarcinoma may be, wherein a biological or biotechnological system is contacted with a soluble substance, such as an oligonucleotide sequence or antibody may be, having affinity with at least one of the genes selected from the group of HGFA, Sema4A, plxnb2, Kng1, FVII, VWF, TFPI, Tna, Plat, serpine2, thrombomodulin and/or their variants and/or parts thereof and/or their mRNA and/or their gene products and/or parts thereof and wherein the soluble substance is linked with a marker.

27. Procedure as claimed in claim 26, wherein the biological or biotechnological system is an organism, a tissue, a cell, a part of a cell, a DNA, a RNA, a cDNA, a mRNA, a cRNA, a protein and/or a peptide and/or a derived structure and/or contains the same.

28. Procedure as according to claim 26-27, wherein the biological or biotechnological system comprises cells of a tumor malignancy of the lung and/or an oligonucleotide library and/or a protein library and/or a peptide library.

29. Procedure according to claim 26-28, wherein the biological system is a transgenic animal, such as a c-myc mouse may be.

30. Use of one or more substances, in particular being identified according to the procedure as claimed in claims 26-29, having affinity with genes selected from the group of HGFA, Sema4A, plxnb2, Kng1, FVII, VWF, TFPI, Tna, Plat, serpine2, thrombomodulin and/or their mutants and/or variations and/or parts thereof and/or their gene products and/or related molecules of said genes and/or derived molecules of said gene products for preparing a medicament for the treatment of a solid adenocarcinoma, in particular of an advanced stage tumor of the lung.

31. Testkit for identifying and/or determining tumor malignancies, in particular advanced stage tumors of the lung, comprising a soluble substance as specified in the claims 26-29.


SEQUENCE LISTING

<110> Fraunhofer-Gesellschaft zur Förderung der angewandten Forschung e.V.

<120> Method for identifying therapeutical targets in tumors, the use of
thereof and means for determining and targeting angiogenesis and hemostasis
related to andenocarcinomas of the lung.

<130> Fra 12 EP

<160> 20

<170> PatentIn version 3.1

<210> 1
<211> 20
<212> DNA
<213> Artificial

<220>
<221> misc_feature
<223> VEGFa forward amplimer

<400> 1
acatcttcaa gccgtcctgt 20

<210> 2
<211> 20
<212> DNA
<213> Artificial

<220>
<221> misc_feature
<223> VEGFa reverse amplimer

<400> 2
gcgagtctgt gtttttgcag 20

<210> 3
<211> 20
<212> DNA
<213> Artificial

<220>
<221> misc_feature
<223> VEGFc forward amplimer

<400> 3
tgtgtccagc gtagatgagc 20

<210> 4
<211> 20
<212> DNA
<213> Artificial

<220>
<221> misc_feature
<223> VEGFc reverse amplimer

<400> 4
tgaggtaacc tgtgctggtg 20

<210> 5
<211> 20
<212> DNA
<213> Artificial

<220>
<221> misc_feature
<223> kdr forward amplimer

<400> 5
cttcctgacc ttggagcatc 20

<210> 6

<211> 20

<212> DNA

<213> Artificial

<220>

<221> misc_feature

<223> kdr reverse amplimer

<400> 6
cagagcaaca caccgaaaga 20

<210> 7

<211> 20

<212> DNA

<213> Artificial

<220>

<221> misc_feature

<223> SPINT-1 forward amplimer

<400> 7
tagcaatggc tgctgtatcg 20

<210> 8

<211> 20

<212> DNA

<213> Artificial

<220>

<221> misc_feature

<223> SPINT-1 reverse amplimer

<400> 8
ccgaagacca aacacatcct 20

<210> 9

<211>  20

<212>  DNA

<213>  Artificial

<220>

<221>  misc_feature

<223>  F7 forward amplimer

<400>  9
ggaacagtgc tcctttgagg                                                    20

<210>  10

<211>  20

<212>  DNA

<213>  Artificial

<220>

<221>  misc_feature

<223>  F7 reverse amplimer

<400>  10
tttgcaggac acctcatctg                                                    20

<210>  11

<211>  20

<212>  DNA

<213>  Artificial

<220>

<221>  misc_feature

<223>  Kng-1 forward amplimer

<400>  11
gaggtgcttg gtcattccat                                                    20

<210>  12

<211>  20

<212>  DNA
<213>  Artificial

<220>
<221>  misc_feature
<223>  Kng-1 reverse amplimer

<400>  12
ctccggaaag gagaaaaacc                                                    20

<210>  13
<211>  20
<212>  DNA
<213>  Artificial

<220>
<221>  misc_feature
<223>  HGF forward amplimer

<400>  13
agaggtccca tggatcacac                                                    20

<210>  14
<211>  20
<212>  DNA
<213>  Artificial

<220>
<221>  misc_feature
<223>  HGF reverse amplimer

<400>  14
gacagggaat tccattccaa                                                    20

<210>  15
<211>  20
<212>  DNA

<213>  Artificial

<220>
<221>  misc_feature
<223>  Met forward amplimer

<400>  15
tgtcagcatc gctcaaattc                                                    20

<210>  16
<211>  22
<212>  DNA
<213>  Artificial

<220>
<221>  misc_feature
<223>  Met reverse amplimer

<400>  16
gtggagactc tctcgcagct ct                                                 22

<210>  17
<211>  20
<212>  DNA
<213>  Artificial

<220>
<221>  misc_feature
<223>  HGFA forward amplimer

<400>  17
gcttcctggg aaatggtaca                                                    20

<210>  18
<211>  20
<212>  DNA
<213>  Artificial

<220>

<221>  misc_feature

<223>  HGFA reverse amplimer


<400>  18
cctcttgcca caggtaggac                                                   20

<210>  19

<211>  20

<212>  DNA

<213>  Artificial


<220>

<221>  misc_feature

<223>  Flt1 forward amplimer


<400>  19
ccctgatggg caaagaataa                                                   20

<210>  20

<211>  20

<212>  DNA

<213>  Artificial


<220>

<221>  misc_feature

<223>  Flt1 reverse amplimer


<400>  20
tccgctgcct tatagatgct                                                   20